(19) Europäisches Patentamt

European Patent Office

Office européen des brevets

(11) Veröffentlichungsnummer: **0 314 904**
**A1**

(12) **EUROPÄISCHE PATENTANMELDUNG**

(21) Anmeldenummer: **88114941.3**

(22) Anmeldetag: **13.09.88**

(51) Int. Cl.4: **C12Q 1/68 , G01N 33/532**

(30) Priorität: **24.09.87 DE 3732145**

(43) Veröffentlichungstag der Anmeldung:
**10.05.89 Patentblatt 89/19**

(84) Benannte Vertragsstaaten:
**BE CH DE FR GB IT LI NL**

(71) Anmelder: **MERCK PATENT GESELLSCHAFT
MIT BESCHRÄNKTER HAFTUNG**
**Frankfurter Strasse 250 Postfach 4119**
**D-6100 Darmstadt(DE)**

(72) Erfinder: **Reckmann, Bernd, Dr.**
**Freiligrathstrasse 7**
**D-610 Seeheim(DE)**
Erfinder: **Rieke, Erwin, Dr.**
**Hermannstrasse 12**
**D-6110 Seeheim(DE)**
Erfinder: **Würzburg, Uwe, Dr.**
**Dessauer Strasse 12**
**D-6110 Dieburg(DE)**
Erfinder: **Renz, Manfred, Dr.**
**Kühler Grund 52/1**
**D-6900 Heidelberg(DE)**
Erfinder: **Czichos, Joachim, Dr.**
**Monchhofstrasse 29**
**D-6900 Heidelberg(DE)**
Erfinder: **Köhler, Martin**
**Konstanzer Strasse 4**
**D-6900 Heidelberg(DE)**

(54) **Verfahren und Reagenz zur Durchführung von Hybridisierungsreaktionen.**

(57) Es betrifft ein Verfahren und Reagenz zur Durchführung von Hybridisierungsreaktionen mit Hilfe eines Komplexes aus einem Polynukleotid und einer bestimmbaren Substanz und Verwendung des so gebildeten Komplexes für Hybridisierungsreaktionen. Das Verfahren ist dadurch gekennzeichnet, daß man ein einzelsträngiges Polynukleotid und eine immunologische Proteinkomponente in Lösung umsetzt und die erhaltene kovalente Polynukleotidverbindung als Sonde in Hybridisierungsreaktionen zum Nachweis von Komplementärsequenzen in fremden Polynukleotiden verwendet.

EP 0 314 904 A1

## Verfahren und Reagenz zur Durchführung von Hybridisierungsreaktionen

Die Erfindung betrifft ein Verfahren und Reagenz zur Durchführung von Hybridisierungsreaktionen mit Hilfe eines Komplexes aus einem Polynukleotid und einer bestimmbaren Substanz.

Die Technik der DNA-Hybridisierung hat in den letzten Jahren zur Erforschung und Diagnose von genetischen und mikrobiologischen Erkrankungen breite Anwendung gefunden. Grundlage der DNA-Hybridisierung ist die Tatsache, daß zwei einzelsträngige komplementäre DNA-Moleküle unter Hybridisierungsbedingungen eine Doppelhelix bilden. In ähnlicher Weise entsteht aus einem einzelsträngigen DNA-Molekül und einem RNA-Molekül ein DNA/RNA-Hybrid.

Zum Nachweis spezifischer Nukleinsäuresequenzen werden die in der Probe enthaltenen Nukleinsäuren durch Denaturierung in Einzelstränge zerlegt. Die entstandenen Einzelstränge werden anschließend auf eine inerte Oberfläche wie Nitrozellulose oder Nylonmembran fixiert. Die fixierten Einzelstränge werden mit einem komplementären Polynukleotid unterhalb der Dissoziationstemperatur in Kontakt gebracht, so daß eine Doppelstrangbildung aus den fixierten Einzelstrangsequenzen und der komplementären Polynukleotidsonde ermöglicht wird. Nach Waschschritten wird der entstandene Doppelstrang aus Polynukleotidsonde und zu bestimmender Nukleinsäuresequenz nachgewiesen.

Zum Nachweis ist die komplementäre Polynukleotidsonde in einer geeigneten Weise für die Wiederauffindung markiert. Üblicherweise erfolgt diese Markierung durch Einbau von Radioisotopen des Phosphors, Jods, Schwefels oder Wasserstoffs.

Ein großer Nachteil dieser Verfahren besteht in der Notwendigkeit des Umgangs mit radioaktiven Substanzen und den damit verbundenen Auflagen und Sicherheitsvorkehrungen. Es sind daher in der Vergangenheit vielfältige Versuche unternommen worden, die radioaktive Markierung von Nukleinsäuren durch nicht-radioaktive Nachweissysteme zu ersetzen.

So wurden z.B. Nukleotidtriphosphat-Analoge synthetisiert, die kovalent gebundenes Biotin an einem Pyrimidin- oder Purinring enthalten (EP 63879). Da diese Nukleotidderivate Substrate für RNA- und DNA-Polymerasen sind, können biotinmarkierte Polynukleotide enzymatisch hergestellt und diese zur Hybridisierung verwendet werden. Der Nachweis beruht auf der seit langem bekannten Avidin-Biotin-Wechselwirkung, wobei die gebildeten Doppelstränge mit einem Affinitätsnachweissystem (z.B. Avidin-Peroxidase) nachgewiesen werden. Dieses Verfahren hat jedoch den Nachteil, daß Biotin in sehr vielen biologischen Materialien vorhanden ist und sein Nachweis daher oft zu Störsignalen führt.

Weiterhin wurde in den letzten Jahren die Antigen-Antikörper-Wechselwirkung zum Nachweis von DNA-Hybridisierungen eingesetzt. Zum Nachweis werden nach erfolgter Hybridisierung klassische immunologische Methoden, wie spezifische, ggf. markierte Antikörper oder Antikörperenzymkonjugate eingesetzt.

Das Antigen in dieser Antigen-Antikörper-Reaktion kann z.B. der gebildete Doppelstrang aus DNA oder DNA/RNA sein. So werden in der EO-OS 135 159 monoklonale Antikörper gegen doppelsträngige native DNA eingesetzt. In der EP-OS 163 220 werden eine Ribonukleinsäure als Sonde und spezifische anti-DNA/RNA-Hybrid-Antikörper eingesetzt. In beiden Fällen erfolgt der Nachweis der zu bestimmenden Nukleinsäure nach erfolgter Hybridisierung durch Bindung der spezifischen markierten Antikörper an die entstandene DNA-Doppelhelices oder die DNA/RNA- oder RNA-/RNA-Hybride.

Der schwerwiegende Nachteil des Einsatzes von spezifischen Antikörpern gegen native DNA liegt darin, daß bei der Hybridisierung der Polynukleotidsonde mit der nachzuweisenden DNA unter Hybridisierungsbedingungen auch Teile der nachzuweisenden DNA Doppelhelices ausbilden und damit zu einer unspezifischen Bindung der Antikörper führen. Ein weiterer Nachteil liegt in der Beschränkung auf Ribonukleinsäuren als Sonden, da die Verwendung von Ribonukleinsäuren besonders enge Bedingungen an die Hybridisierungsreaktion stellt, um eine Schädigung oder Zerstörung der Ribonukleinsäure durch einen zu hohen pH-Wert oder eingeschleppte Ribonuklease-Aktivität zu verhindern.

Das Antigen in der immunologischen Reaktion kann auch eine Polynukleotidsonde sein, die mit einem Hapten kovalent modifiziert ist. Dabei wird das Hapten über ein Brückenglied kovalent mit der Polynukleotidsonde verbunden. Ein möglicher Nachteil dieser Methode liegt darin, daß das Hapten eine effiziente Hybridisierung zwischen der Polynukleotidsonde und der nachzuweisenden DNA stören kann.

Der Erfindung liegt die Aufgabe zugrunde, ein neues Verfahren und Reagenz zur Durchführung von Hybridisierungsreaktionen zum Nachweis von Komplementärsequenzen zu schaffen, mit dem die oben erwähnten Nachteile vermieden werden können.

Gelöst wird diese Aufgabe erfindungsgemäß durch ein Verfahren zur Durchführung von Hybridisierungsreaktionen durch Komplexbildung zwischen einem Polynukleotid und einer bestimmbaren Substanz und Verwendung des so gebildeten Komplexes für Hybridisierungsreaktionen, das dadurch gekennzeichnet ist, daß man ein einzelsträngiges Polynukleotid und eine immunologische Proteinkomponente in Lösung umsetzt und die erhaltene kovalente Polynukleotidverbindung als Sonde in Hybridisierungsreaktionen zum Nachweis von Komplementärsequenzen in fremden Polynukleotiden verwendet.

Ein weiterer Gegenstand der Erfindung ist ein Reagenz zur Durchführung dieses Verfahrens, das dadurch gekennzeichnet ist, daß es aus einem kovalent vernetzten Komplex aus einem einzelsträngigen Polynukleotid und einer immunologischen Proteinkomponente besteht oder diesen enthält.

Das erfindungsgemäße Verfahren beruht auf der Beobachtung, daß Konjugate aus einer immunologischen Proteinkomponente und einzelsträngigen Nukleinsäuren als Hybridisierungssonden verwendbar sind. Die eingesetzte Nukleinsäure kann naturlichen oder synthetischen Ursprungs sein.

Zur Kopplung wird die Nukleinsäure gegebenenfalls durch Denaturierung in ihre Einzelstränge aufgetrennt.

Als immunologische Proteinkomponenten eignen sich im Rahmen der Erfindung Protein A, Immunoglobuline, Antikörperfragmente, Protein G und andere Proteine, die mit Antikörpern interagieren, vorzugsweise Protein A und Immunoglobuline.

Zur Sichtbarmachung des Hybridisierungsproduktes muß die Proteinkomponente vor oder nach der Komplexbildung markiert werden. Die Durchführung der Markierung erfolgt nach den hierfür bekannten Methoden der Proteinchemie. Als Markierungssystem kommt sowohl eine Markierung mit biologisch aktiven Proteinen, z.B. Enzymen, als auch eine Markierung mit Farbstoffen, Farbstoffkomponenten oder radioaktiven Substanzen in Betracht. Bevorzugte Markierungen nach der Erfindung sind solche mit Enzymen wie Peroxidase, ß-Galaktosidase, alkalische Phosphatase usw. Anstelle einer Markierung der Proteinkomponente ist es auch möglich, das Protein durch eine immunologische Reaktion nachzuweisen, wobei der Immunreaktionspartner in diesem Falle markiert sein muß oder mit einem zweiten Antikörper nachgewiesen wird. Die bestimmbaren Gruppen als solche können entweder direkt bestimmbar sein wie bei radioaktiven- oder Farbstoffmarkierungen, oder indirekt, z.B. aufgrund ihrer enzymatischen Aktivität, wie dies aus dem Enzym-Immuno-Assay-Verfahren bekannt ist.

Die Herstellung des Komplexes zwischen dem Polynukleotid und der Proteinkomponente erfolgt durch UV-Bestrahlung im sauren pH-Bereich, vorzugsweise im Bereich von pH 1-3, insbesondere im Bereich von pH 1,5-2,5. Aus der EP-PS 131 830 ist zwar schon ein Verfahren zur Markierung von Nukleinsäuren bekannt, wobei die Nukleinsäure mit speziellen photochemisch-reaktiven Nukleinsäurebindenden Liganden in Berührung gebracht und bestrahlt wird. Diese Umsetzung erfolgt im neutralen pH-Bereich und erfordert etwa eine Stunde. Überraschenderweise erhält man nach dem erfindungsgemäßen Verfahren im stark sauren pH-Bereich ohne weitere Hilfsmittel innerhalb weniger Minuten eine kovalente Bindung zwischen der Nukleinsäure und dem Protein. Pro 200 Nukleinsäureeinheiten wird etwa ein Proteinmolekül kovalent gebunden.

Neben der photochemischen Methode können zur Herstellung von Nukleinsäure-IgG-Konjugaten auch bifunktionelle Vernetzer verwendet werden. Solche Vernetzer sind allgemein bekannt und umfassen z.B. Dialdehyde wie Glutardialdehyd, Dicarbonsäuren, Diester, Dianhydride, Diamine oder auch eine Kombination solcher Gruppen. Eine Steigerung der Hybridisierungseffizienz solcher Konjugate wird überraschenderweise erreicht, wenn zu deren Synthese an Stelle von Gesamt-IgG eine durch DEAE-Zellulose-Chromatographie isolierte IgG-Fraktion eingesetzt wird. Die synthetisierten Konjugate sind stabil gegenüber den normalen Hybridisierungsbedingungen (50 % Formamid, 40 $^\circ$C). Darüberhinaus wird durch die Proteinbindung die Hybridisierungseffizienz der gekoppelten Nukleinsäure nicht beeinträchtigt. Nukleinsäure-Protein A-Konjugate zeichnen sich durch eine besonders hohe Thermostabilität aus, so daß sie auch in Hybridisierungsreaktionen, die bei 65 $^\circ$C durchgeführt werden, eingesezt werden können.

Nach erfolgter Hybridisierung wird das gekoppelte immunologische Protein direkt mittels bekannter Verfahren nachgewiesen, z.B. mit Enzymoder Biotin-markierten Antikörpern. Außerdem sind andere Detektionssysteme anwendbar, indem durch Ausnutzung freier Antigenbindungsstellen der Immunoglobuline entsprechende Antigene (z.B. Enzyme) gebunden werden können. Hierdurch eröffnet sich eine große Anzahl bekannter Nachweismethoden, die in heterogener oder homogener Verfahrensweise an den Hybridisierungsschritt angefügt werden können.

Der Vorteil des erfindungsgemäßen Verfahrens liegt darin, daß durch eine einfache, schnelle und durch geringen Aufwand gekennzeichnete Methode zwei Makromoleküle (DNA/RNA und ein immunologisches Protein) mit unterschiedlichen spezifischen Bindungseigenschaften aneinander gekoppelt werden. Die Bindungsspezifität der Nukleinsäure-Komponente dient der Erkennung des Analyten (DNA/RNA). Die IgG- bzw. Antigen-Bindungseigenschaften der Proteinkomponenten werden als Teil des Detektorsystems ausgenutzt. Auf diese Weise lassen sich fremde Nukleinsäuren sehr empfindlich nachweisen. Es wurden Analysenergebnisse erzielt, die den herkömmlichen, nicht-radioaktiven Methoden mindestens ebenbürtig sind. Eine weitere Verwendungsmöglichkeit spezieller Nukleinsäure-IgG- bzw. Nukleinsäure-Protein A-Konjugate besteht darin, diese zum Antigen-Nachweis einzusetzen (ELISA).

Beispiel 1
---

Synthese von DNA-Protein A-Konjugaten

Der Reaktionsansatz (Endvolumen 100 µl) enthält 1 µg beschallte Plasmid-DNA und 5 µg Protein A aus Staphylococcus aureus in 50 mM Natriumcitrat, pH 2,5; unter diesen Bedingungen liegt die DNA in einzelsträngiger Form vor. Die Bestrahlung erfolgt in einem 1,5-ml-Plastikgefäß mit einer schwachen UV-Lampe (Hg-Niederdurck-Röhre, 8 Watt, 254-nm-Filter) aus 7 cm Entfernung (10 Min., 0 °C). Nach Neutralisation mi 12 µl 1 N NaOH wird ein Aliquot des Ansatzes direkt zur Hybridisierung verwendet.

Beispiel 2
---

Hybridisierung mit DNA-Protein A-Konjugaten bei 37 °C

Die zu untersuchende DNA wird entweder mit Restriktionsendonucleasen gespalten, durch Agarosegelelektrophorese aufgetrennt und nach der Southern-Methode (J. Mol. Biol. 98, 503-517, 1975) auf Nitrozellulose-Papier übertragen ("Southern-Blots") oder nach Hitze-denaturierung in ver schiedenen Verdünnungen direkt punktförmig auf Nitrozellulose aufgetragen ("Dot-Blots"). Die Papiere werden in Plastikschalen 30 Min. bei 37 °C in 10 x Denhardt's Losung (Biochem.Biophys.Res.comm. 23, 641-645, 1966; 0,1 % SDS; 4 x SET eingeweicht (1 x SET = 0,15 M NaCl; 0,02 M Tris-HCl pH 8; 1 mM EDTA). Anschließend erfolgt eine Vorinkubation für 30-60 Min. bei 37 °C in 50 % deionisiertem Formamid; 6 % Polyethylenglykol; 2 x Denhardt's-Losung; 4 x SET; 0,1 % SDS und 100 µg/ml Hefe-t-RNA. Nach Zugabe der Probe (50 ng DNA der nach Beispiel 1 hergestellten Konjugate pro 1 ml Hybridisierungslösung) wird unter leichtem Schütteln bei 37 °C 16-20 Stunden hybridisiert. Die Nitrozellulose-Papiere werden dann 2 x 30 Min. bei 37 °C in 50 % Formamid; 0,2 % SDS; 2 x SSC (1 x SSC = 0,15 M NaCl; 0,015 M Natriumcitrat) und anschließend 2 x 10 Min. bei 20 °C in 2 x SSC gewaschen.

Beispiel 3
---

Hybridisierung mit DNA-Protein A-Konjugaten bei 65 °C

Die Hybridisierung unterscheidet sich von dem in Beispiel 2 dargestellten Verfahren durch die Hybridisierungstemperatur und die Zusammensetzung der Hybridisierungslösung. Die Papiere mit der zu testenden DNA werden bei 65 °C in folgender, DNA-Protein A-Konjugat enthaltender Lösung für 16 Stunden inkubiert: 4 x SET; 2 x Denhardt's; 0,1 % SDS; 50 µg/ml t-RNA. Danach wird 2 x 20 Min. bei 65 °C in 2 x SET; 0,1 % SDS und anschließend 2 x 5 Min. bei 20 °C in 2 x SET gewaschen.

Beispiel 4
---

Immunologischer Nachweis von DNA-Protein A-Konjugaten

Die Nitrozellulose-Papiere werden für 20 Min. bei 37 °C in einer Losung aus 3 % BSA (Rinderserumalbumin) in Puffer A (150 mM NaCl; 10 mM Tris-HCl pH 7,4; 5 mM EDTA und 0,1 % Triton X 100) inkubiert. Danach werden die Papiere in eine Lösung aus 0,3 % BSA in Puffer A überführt, die Peroxidasekonjugiertes IgG aus Kaninchen enthält, und 30 Min. bei 20 °C unter leichtem Schütteln darin belassen. Anschließend erfolgen 3 Waschschritte von je 5 Min. bei 20 °C in 0,3 % BSA/puffer A. Um die gebildeten Hybride sichtbar zu machen, werden die Papiere in eine Farblösung überführt, die sich aus 10 ml 100 mM Tris-Hcl pH 7,4; 10 mM Imidazol und 2 ml Ethanol, worin 6 mg 3,3'-Dianisidin gelöst werden, und 10 µl 30 % Wasserstoffperoxid $H_2O_2$ zusammensetzte. Die Farbstoffentwicklung erfolgt während 5-30 Min. bei 20 °C im Dunkeln.

Alternativ kann folgendes, sensivere Nachweissystem angewandt werden: Nach Inkubation in 3 % BSA / Puffer A wird in die gleiche Lösung anti-Protein A-Antiserum (Verdünnung 1 : 2000) aus Kaninchen gegeben (30 Min., 20 °C). Dann erfolgen 3 Waschschritte von je 5 Min. in 0,3 % BSA / Puffer A. Die Nitrozellulose-Papiere werden dann 30 - 60 Min. mit Peroxidase-gekoppeltem anti-Kaninchen-IgG-Antikörper aus Ziege inkubiert. Dann wird 3 x 5 Min. gewaschen und die Farbentwicklung durchgeführt.

Beispiel 5
---

Nachweis der Hybridisierung von DNA-Protein A-Konjugaten durch einen gegen Peroxidase gerichteten Antikörper

Wie in Beispiel 4 werden die Papiere in 3 % BSA/Puffer A für 20 Min. bei 37 °C inkubiert. Danach werden die Papiere in eine 0,3 % BSA/Puffer A-Lösung mit Kaninchen-anti-Peroxidase IgG überführt und 30 Min. bei 20 °C inkubiert. Nach 3 Waschschritten von je 5 Min. bei 20 °C mit 0,3 % BSA/Puffer A werden die Filter in derselben Lösung für 30 Min. bei 20 °C mit Meerrettich-Peroxidase (100 µg/µl, 1:100 verdünnt) inkubiert. Danach erfolgen 3 Waschschritte mit 0,3 % BSA/Puffer A, bevor die Papiere in die Färbelösung (entsprechend Beispiel 4) überführt werden.

Beispiel 6

Synthese von DNA-IgG-Konjugaten durch Glutardialdehyd

Der Reaktionsansatz (Endvolumen 40 µl) enthält 1 µg beschallte, hitzedenaturierte Plasmid-DNA, 80 µg Immunglobulin G aus Kaninchen und 5 % Glutardialdehyd in 4 mM Natriumphosphat-Puffer pH 6,8. Nach Inkubation bei 37 °C für 7,5 Min. erfolgt eine Auftrennung des Reaktionsgemisches mit Hilfe einer Sepharose Cl-6B-Säule (6 ml, 15 cm). Zur Elution wird 10 mM Tris-HCl pH 7,4; 5 mM EDTA verwendet (Fraktionsgröße 500 µl). Die ersten DNA-haltigen Fraktionen (ca. 20 % der Gesamt-DNA) enthielten Konjugate, die zur Hybridisierung eingesetzt wurden.

Die Ausbeute und Hybridisierungseffizienz an DNA-IgG-Konjugaten können erhöht werden, wenn anstelle von Gesamt-IgG eine durch DEAE-Cellulose-Chromatographie gewonnene Fraktion verwendet wird. Gesamt-IgG wird aus Kaninchen-Serum durch Affinitätschromatographie (Protein A-Säule) oder Ammoniumsulfat-Fällung isoliert und nach Dialyse gegen 10 mM Kaliumphosphat, pH 7,2, einer DEAE-Säulen-Chromatographie unterworfen. Die im Durchlauf eluierende Fraktion (ca. 40 % der Gesamt-IgG's) wird zur Herstellung der Konjugate verwendet.

Beispiel 7

Synthese von DNA-IgG-Konjugaten durch UV-Bestrahlung

Der Reaktionsansatz (Endvolumen 100 µl) enthält 1 µg beschallte, hitzedenaturierte Plasmid-DNA und 3 µg Immunglobulin G aus Kaninchen in 50 mM Natrium-Citrat pH 2,5. Die weitere Vorgehensweise erfolgt wie in Beispiel 1 beschrieben.

Beispiel 8

Hybridisierung mit DNA-IgG-Konjugaten

Die Hybridisierung von "Southern-Blots" und "Dot-Blots" erfolgt wie in Beispiel 2 beschrieben. 1 ml Hybridisierungslösung enthält 100 ng DNA der nach Beispiel 6 oder 7 hergestellten Konjugate.

Beispiel 9

Nachweis der Hybridisierung von DNA-IgG-Konjugaten

Der Nachweis der Hybride erfolgt wie im Beispiel 4 beschrieben, jedoch wird an Stelle von Peroxidase-konjugiertem Kaninchen-IgG Peroxidase-konjugiertes Anti-Kaninchen-IgG aus Ziege verwendet.

**Ansprüche**

1. Verfahren zur Durchführung von Hybridisierungsreaktionen durch Komplexbildung zwischen einem Polynukleotid und einer bestimmbaren Substanz und Verwendung des so gebildeten Komplexes für Hybridisierungsreaktionen, dadurch gekennzeichnet, daß man ein einzelsträngiges Polynukleotid und eine immunologische Proteinkomponente in Lösung umsetzt und die erhaltene kovalente Polynukleotidverbindung als Sonde in Hybridisierungsreaktionen zum Nachweis von Komplementärsequenzen in fremden Polynukleotiden verwendet.

2. Verfahren nach Anspruch 1, dadurch gekennzeichnet, daß als immunologische Proteinkomponente Protein A oder Immunoglobuline verwendet werden.

3. Verfahren nach den Ansprüchen 1 und 2, dadurch gekennzeichnet, daß man die Proteinkomponente vor oder nach der Komplexbildung markiert.

4. Verfahren nach den Ansprüchen 1 bis 3, dadurch gekennzeichnet, daß der Komplex durch UV-Bestrahlung im sauren pH-Bereich hergestellt wird.

5. Verfahren nach den Ansprüchen 1 bis 3, dadurch gekennzeichnet, daß der Komplex mit Hilfe von bifunktionellen Vernetzern hergestellt wird.

6. Reagenz zur Durchführung des Verfahrens nach den Ansprüchen 1-5, dadurch gekennzeichnet, daß es aus einem kovalent vernetzten Komplex aus einem einzelsträngigen Polynukleotid und einer immunologischen Proteinkomponente besteht oder diesen enthält.

7. Reagenz nach Anspruch 6, dadurch gekennzeichnet, daß es als Proteinkomponente Protein A oder Immunoglobuline enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 019 408 (INSTITUT PASTEUR) * Seite 4, Zeilen 44-51; Seite 5, Zeile 22 - Seite 6, Zeile 3 * --- | 1,3,6,7 | C 12 Q 1/68 G 01 N 33/532 |
| X | US-A-4 358 535 (S. FALKOW et al.) * Spalte 1, Zeile 64 - Spalte 2, Zeile 17; Spalte 2, Zeilen 43-44; Spalte 3, Zeile 28 - Spalte 4, Zeile 18 * --- | 1,3,6,7 | |
| X | WO-A-8 403 520 (A.D.B. MALCOLM et al.) * Seite 3, Zeilen 22-32; Seite 5, Zeile 21 - Seite 6, Zeile 11; Seite 8, Zeilen 23-33; Seite 12, Zeile 23 - Seite 13, Zeile 10; Seite 24, Zeile 1 - Seite 25, Zeile 19 * --- | 1,3,6,7 | |
| X,D | EP-A-0 131 830 (MOLECULAR DIAGNOSTICS) * Seite 8, Zeile 19 - Seite 9, Zeile 23; Seite 16, Beispiel 4; Seite 22, Zeilen 2-5,15-18 * --- | 1,3,6,7 | |
| X | GB-A-2 125 964 (CETUS MADISON CORP.) * Seite 1, Zeilen 76-95; Seite 3, Zeilen 81-83,97-112; Seite 10, Zeilen 13-30 * --- | 1,3,6,7 | **RECHERCHIERTE SACHGEBIETE (Int. Cl.4)** <br><br> C 12 Q 1 <br> G 01 N 33 |
| X | US-A-4 587 044 (P.S. MILLER et al.) * Spalte 1, Zeilen 42-49; Spalte 2, Zeile 60 - Spalte 3, Zeile 60; Spalte 21, Zeile 64 - Spalte 24, Zeile 14 * --- -/- | 1,3,6,7 | |

Der vorliegende Recherchenbericht wurde für alle Patentansprüche erstellt

| Recherchenort | Abschlußdatum der Recherche | Prüfer |
|---|---|---|
| DEN HAAG | 16-12-1988 | VAN BOHEMEN C.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
                     
& : Mitglied der gleichen Patentfamilie, übereinstimmendes Dokument

EPO FORM 1503 03.82 (P0403)

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 154 884 (MOLECULAR DIAGNOSTICS) * Seite 4, Zeilen 10-26; Seite 5, Zeile 12 - Seite 6, Zeile 18; Seite 10, Beispiel; Seite 11, Beispiel 2; Seite 12, Beispiel 3; Seite 13, Beispiel 6; Seite 14, Beispiel 7; Seite 16, Zeile 1 - Seite 17, Zeile 28 * --- | 1-3,5-7 | |
| X | CHEMICAL ABSTRACTS, Band 88, 1978, Seite 219, Nr. 132899b, Columbus, Ohio, US; M. KROEGER et al.: "Nucleophilic substitution at C-2 of S-alkylated 2-thiocytidines by cycteine and lysine. A new method for specific covalent linking of peptides to nucleic acids", & BIOORG. CHEM. 1977, 6(4), 431-41 * Abstract * --- | 1,3,6,7 | |
| X | CHEMICAL ABSTRACTS, Band 93, 1980, Seite 399, Nr. 64767h, Columbus, Ohio, US; Y.-C. TSE et al.: "Covalent bonds between protein and DNA. Formation of phosphotyrosine linkage between certain DNA topoisomerases and DNA" & J. BIOL. CHEM. 1980, 255 (12), 5560-5 * Abstract * --- | 1,3,6,7 | |
| X | CHEMICAL ABSTRACTS, Band 98, 1983, Seite 229, Nr. 157399h, Columbus, Ohio, US; S. CHENG et al.: "A versatile method for the coupling of protein to DNA: synthesis of alpha2-macroglobulin-DNA conjugates", & NUCLEIC ACIDS RES. 1983, 11(3), 659-69 * Abstract * ----- | 1,3,6,7 | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 16-12-1988 | VAN BOHEMEN C.G. |